(19) Europäisches Patentamt
European Patent Office
Office européen des brevets

(11) Numéro de publication : **0 604 259 A1**

# (12) DEMANDE DE BREVET EUROPEEN

(21) Numéro de dépôt : **93402949.7**

(51) Int. Cl.$^5$ : **C07D 401/14**, A61K 31/47

(22) Date de dépôt : **07.12.93**

(30) Priorité : **14.12.92 FR 9215038**

(43) Date de publication de la demande : **29.06.94 Bulletin 94/26**

(84) Etats contractants désignés : **AT BE CH DE DK ES FR GB GR IE IT LI LU MC NL PT SE**

(71) Demandeur : **SYNTHELABO**
**22, Avenue Galilée**
**F-92350 Le Plessis Robinson (FR)**

(72) Inventeur : **Cremer, Gérard**
**35 rue de Sables**
**F-91420 Morangis (FR)**
Inventeur : **Muller, Jean-Claude**
**4, Avenue de l' Espérance**
**F-93390 Morsang-sur-Orge (FR)**

(74) Mandataire : **Thouret-Lemaitre, Elisabeth et al**
**SYNTHELABO,**
**Service Brevets,**
**B.P. 72**
**F-92352 Le Plessis-Robinson Cédex (FR)**

## (54) Dérivés de 3-(quinoléin-6-yl-méthyl)-4H-imidazol-4-one comme angiotensin II antagonistes.

(57) Dérivés de 3-(quinoléin-6-yl-méthyl)-4*H*-imidazol-4-one répondant à la formule (I)

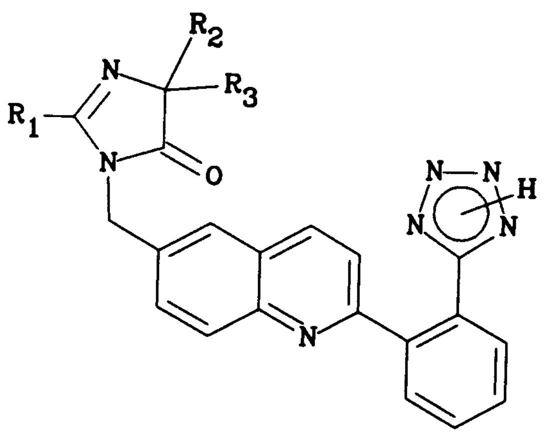

(I)

dans laquelle
$R_1$ représente un groupe $(C_2\text{-}C_5)$alkyle droit ou ramifié,
$R_2$ et $R_3$ représentent, chacun indépendamment l'un de l'autre, soit un atome d'hydrogène, soit un groupe $(C_1\text{-}C_5)$alkyle droit ou ramifié, soit un groupe $(CH_2)_n$-aryle où n = 0 à 3,
ou $R_2$ et $R_3$ peuvent former avec le cycle imidazole un groupe spirocyclo$(C_3\text{-}C_8)$ alkyle,
ainsi que leurs sels d'addition aux acides et aux bases pharmaceutiquement acceptables,
Application thérapeutique.

EP 0 604 259 A1

La présente invention a pour objet des dérivés de 3-(quinoléin-6-yl-méthyl)-4*H*-imidazol-4-one, leur préparation et leur application en thérapeutique.

Les composés de l'invention répondent à la formule (I)

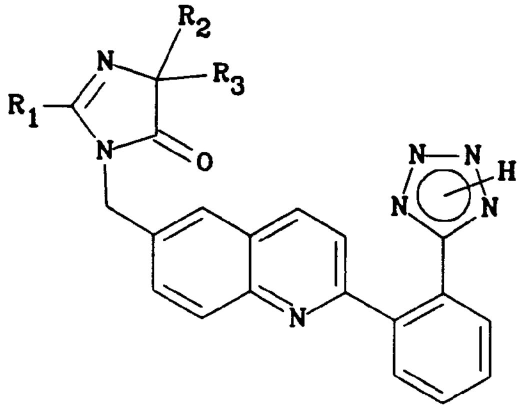

(I)

dans laquelle

$R_1$ représente un groupe $(C_2-C_5)$alkyle droit ou ramifié,

$R_2$ et $R_3$ représentent, chacun indépendamment l'un de l'autre, soit un atome d'hydrogène, soit un groupe $(C_1-C_5)$alkyle droit ou ramifié, soit un groupe $(CH_2)_n$-aryle où n = 0 à 3,

ou $R_2$ et $R_3$ peuvent former avec le cycle imidazole un groupe spirocyclo$(C_3-C_8)$alkyle.

Les composés préférés de l'invention sont ceux pour lesquels

$R_1$ représente un groupe $(C_2-C_5)$alkyle droit ou ramifié,

$R_2$ et $R_3$ représentent, chacun indépendamment l'un de l'autre, un groupe $(C_1-C_5)$alkyle droit ou ramifié,

ou $R_2$ et $R_3$ peuvent former avec le cycle imidazole un groupe spirocyclo$(C_3-C_8)$alkyle.

Parmi ceux-ci les composés de choix selon l'invention sont ceux pour lesquels

$R_1$ représente un groupe butyle,

$R_2$ et $R_3$ représentent, chacun indépendamment l'un de l'autre, un groupe $(C_1-C_5)$alkyle droit ou ramifié,

ou $R_2$ et $R_3$ peuvent former avec le cycle imidazole un groupe spirocyclo$(C_3-C_8)$alkyle.

Les composés de l'invention peuvent se présenter sous forme libre ou sous forme de sels organiques ou inorganiques pharmaceutiquement acceptables.

Conformément à l'invention, les composés de formule (I) peuvent être synthétisés selon le schéma 1 de la page suivante.

On fait réagir à la température de reflux, la 4-méthylbenzènamine (paratoluidine) avec un benzaldéhyde de formule (II), dans laquelle X représente un atome de brome ou d'iode, en présence d'un catalyseur tel l'acide 4-méthylbenzènesulfonique (acide paratoluènesulfonique), en solution dans du benzène. Après refroidissement, on ajoute de l'acide propiolique et l'on chauffe à la température de reflux de manière à obtenir le composé de formule (III). Ensuite, on chauffe un mélange du composé de formule (III) et de cyanure cuivreux dans un solvant tel que la pyridine, de manière à obtenir le 2-(6-méthylquinoléin-2-yl)benzonitrile (IV) que l'on fait réagir avec un azidure organométallique tel l'azidure de triméthylétain, ou un azidure métallique tel l'azidure de sodium, de manière à obtenir un composé sur lequel on fait passer un courant d'acide chlorhydrique gazeux, pour obtenir la quinoléine de formule (V). La première réaction est conduite dans un solvant tel que le xylène à la température de reflux; la seconde réaction est conduite dans un solvant tel que le mélange toluène/tétrahydrofurane, à la température ambiante.

Puis on protège, par un groupe protecteur $R_4$, où $R_4$ représente un groupe de formule $CR_5R_6R_7$ dans laquelle $R_5$, $R_6$ et $R_7$ sont chacun indépendamment l'un de l'autre un groupe $(C_1-C_2)$alkyle ou un groupe aryle, le groupe tétrazole de la quinoléine de formule (V); dans cette étape, on fait réagir le composé (V) avec un agent protecteur, tel par exemple le chlorure de trityle à la température ambiante dans un solvant tel que le dichlorométhane, en présence d'une base telle que la N-méthylmorpholine ou la triéthylamine et on obtient un composé de formule (VI) dans laquelle $R_5$, $R_6$ et $R_7$ sont tels que définis précédemment. Ensuite, on fonctionnalise le groupe méthyle en position 6 de la quinoléine de formule (VI), en y

## Schéma 1

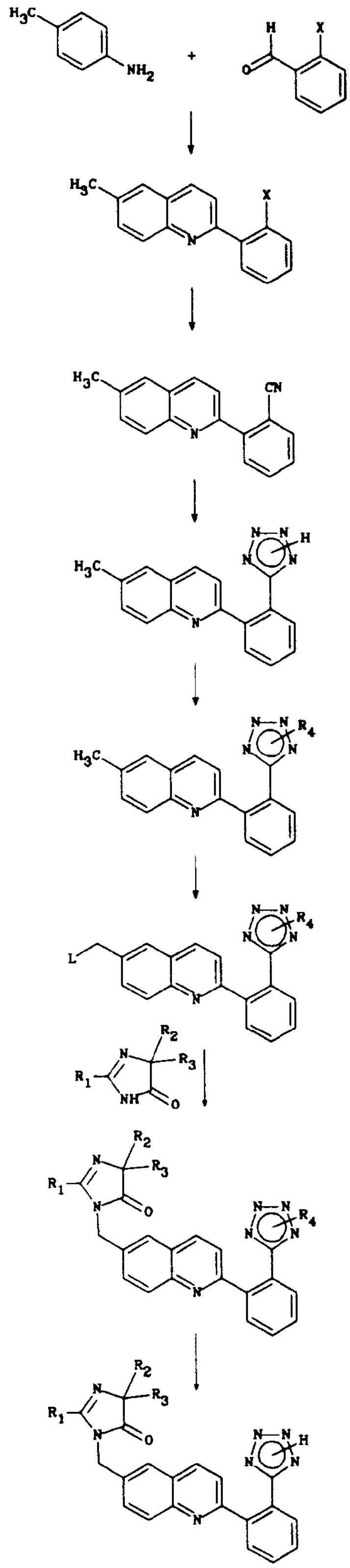

(II)

(III)

(IV)

(V)

(VI)

(VII)

(VIII)

(IX)

(I)

introduisant un groupe partant L, par exemple un radical bromo et on obtient un composé de formule (VII) où $R_5$, $R_6$ et $R_7$ sont tels que définis précédemment; la réaction est conduite à la température de reflux dans un solvant tel que le tétrachlorure de carbone, en présence d'un initiateur tel que le peroxyde de benzoyle ou l'α,α'-azobisisobutyronitrile, à la température de reflux. Finalement, on condense, dans le diméthylformamide, à une température de 0 °C à 50 °C, en présence d'une base telle que l'hydroxyde de potassium ou le carbonate de potassium, le composé de formule (VII) avec une imidazolone de formule (VIII) dans laquelle $R_1$, $R_2$ et $R_3$ sont tels que définis précédemment et on obtient un composé de formule (IX) que l'on soumet à une déprotection.

Les composés de formule (IX)

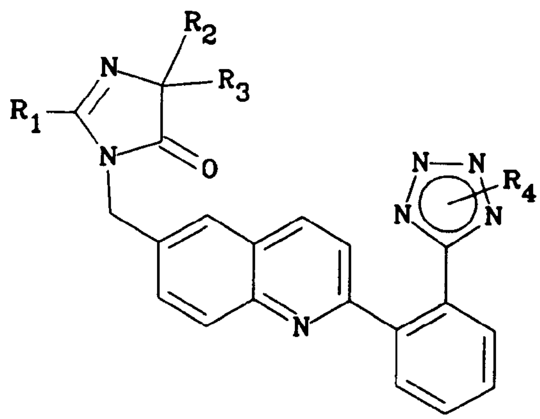

(IX)

dans laquelle $R_1$, $R_2$, $R_3$ et $R_4$ sont tels que définis ci-dessus sont nouveaux et font partie de l'invention.

Les composés de départ sont disponibles dans le commerce ou décrits dans la littérature ou peuvent être préparés selon des méthodes qui y sont décrites ou qui sont connues de l'homme du métier.

Ainsi les imidazolones de formule (VIII) peuvent être synthétisées selon la méthode décrite par Jacquier et coll. (Bull. Soc. Chim. France, 1971, 3, 1040-1051), par condensation d'un imidate d'alkyle et d'un ester d'acide aminé, tel que par exemple le 1-amino-1-cyclopropylcarboxylate de méthyle.

Le 1-amino-1-cyclopropylcarboxylate de méthyle est préparé selon la méthode décrite par Valdyanathan (J. Org.Chem., 1989, 54, 1810-1815).

Les composés de formule (VII) sont préparés selon la méthode décrite dans la demande de brevet européen n° 0540500 de la demanderesse.

Les exemples suivants illustrent l'invention.

Les microanalyses et les spectres IR et RMN confirment la structure des composés obtenus.

## Exemple 1

5-butyl-6-[[2-[2-(1*H*-tétrazol-5-yl]phényl)quinoléin-6-yl]méthyl]-4,6-diazaspiro[2.4]hept-4-èn-7-one

1.1. 6-bromométhyl-2-[2-[1(ou 2)-(triphénylméthyl)-1(ou 2)*H*-tétrazol-5-yl]phényl]quinoléine

1.1.1. 2-(2-bromophényl)-6-méthylquinoléine

On chauffe à la température de reflux, dans un ballon surmonté d'un Dean-Stark, 50 g (270 mmoles) de 2-bromobenzaldéhyde avec 29,5 g (276 mmoles) de paratoluidine et 0,5 g d'acide paratoluènesulfonique en solution dans un litre de benzène. Lorsque l'élimination d'eau est terminée (environ 5 ml), on ajoute au milieu réactionnel préalablement refroidi vers 50 °C, 8,3 ml (135 mmoles) d'acide propiolique. On note un dégagement important de $CO_2$ et on porte au reflux pendant 3 heures. On contrôle la réaction par chromatographie sur couche mince, dans un mélange de dichlorométhane et d'hexane (70/30). Dans ces conditions expérimentales, il a été nécessaire d'ajouter un excès de 20% d'acide propiolique suivi d'un reflux d'1 heure pour parfaire la réaction. On évapore le solvant sous pression réduite et on purifie le résidu par chromatographie sur colonne de silice, en éluant avec un mélange de dichlorométhane et d'hexane (70/30).

On récupère 22 g du dérivé attendu, sous forme d'un composé cristallisé.

M = 22 g

RMN $^1H$ (200 MHz, $CDCl_3$): d 2,55 (s, 3H), 7,25-7,70 (m, 7H), 8,02-8,15 (m, 2H).

De la même manière, on prépare la 2-(2-iodophényl)-6-méthyl-quinoléine à partir du 2-iodobenzaldéhyde.

F = 77-77,5 °C

1.1.2. 2-(6-méthylquinoléin-2-yl)benzonitrile

On chauffe à 160 °C pendant 12 heures, sous argon, un mélange contenant, 15 g (50 mmoles) du composé précédemment obtenu en 1.1.1 et 5 g (56 mmoles) de cyanure cuivreux dans 60 ml de pyridine. On contrôle la réaction par chromatographie sur couche mince dans du dichlorométhane. On évapore la pyridine sous pression réduite et on reprend le résidu par du dichlorométhane. On lave la phase organique plusieurs fois avec une solution aqueuse d'ammoniaque, jusqu'à ce que la phase aqueuse soit incolore. Après un dernier lavage à l'eau, on sèche la phase organique sur du sulfate de magnésium et on évapore le solvant. On reprend le résidu par de l'éther de pétrole.

M = 9,6 g F = 157 °C Rdt = 78 %

1.1.3. 6-méthyl-2-[2-(1*H*-tétrazol-5-yl)phényl]quinoléine, chlorhydrate

Dans 110 ml de xylène on introduit, 9,6 g (39,3 mmoles) du nitrile obtenu précédemment en 1.1.2 et 14,96 g (72,7 mmoles) d'azidure de triméthylétain. On porte ce mélange au reflux pendant 15 heures. Après refroidissement, on filtre le solide et on le met en suspension dans 115 ml de toluène et 7 ml de tétrahydrofurane. On soumet le mélange, refroidi par un bain de glace, à un barbotage de gaz chlorhydrique pendant 2 heures. On récupère la fraction insoluble par filtration puis on la lave avec du toluène et avec de l'eau.

M = 13 g

1.1.4. 6-méthyl-2-[2-[1(ou 2)-(triphénylméthyl)-1(ou 2)*H*-tétrazol-5-yl]phényl]quinoléine

Dans un litre de dichlorométhane et à température ambiante, on ajoute, 80,5 g (219 mmoles) du composé obtenu précédemment en 1.1.3, 60 ml (547 mmoles) de N-méthyl morpholine et 73,1 g (262 mmoles) de chlorure de trityle. On agite la solution pendant une nuit, on reprend par de l'eau, on lave la phase organique deux fois par de l'eau puis on sèche. On évapore le solvant et on cristallise le résidu dans un minimum d'éther.

M = 119 g F = 176-177 °C Rdt = 87 %

1.1.5. 6-bromométhyl-2-[2-[1(ou 2)-(triphénylméthyl)-1(ou 2)*H*-tétrazol-5-yl]phényl]quinoléine

Dans 300 ml de tétrachlorure de carbone on ajoute 10 g (189 mmoles) du composé précédemment obtenu en 1.1.4 et on porte le mélange vers 60 °C jusqu'à dissolution totale. A cette température, on ajoute en une fois 3,7 g (20,8 mmoles) de N-bromosuccinimide et 60 mg (0,37 mmole) d'$\alpha,\alpha'$-azobisisobutyronitrile. On porte le mélange à température de reflux pendant 2 à 3 heures, jusqu'à disparition du N-bromosuccinimide. Au mélange refroidi, on ajoute 100 ml d'eau et 300 ml de dichlorométhane. On lave la phase organique plusieurs fois à l'eau, puis on la sèche. On évapore le solvant et on triture le résidu dans du diisopropyléther. On obtient un produit pur à 90 % qu'on utilisera tel quel.

M = 10,3 g

1.2. chlorhydrate de 5-butyl-4,6-diazaspiro[2.4]hept-4-èn-7-one

On chauffe à la température de reflux, pendant 8 heures, un mélange de 15,6 g (121 mmoles) de pentanimidate d'éthyle et de 13,5 g (117,4 mmoles) de 1-amino-1-cyclopropylcarboxylate de méthyle dans 150 ml de xylène auxquels on ajoute 20 gouttes d'acide acétique. On évapore le solvant, on reprend le résidu par de l'éther et on acidifie par de l'acide chlorhydrique 12 N. On obtient un précipité que l'on filtre.

RMN $^1$H, 200 MHz($CDCl_3$) d 0,95 (t, 3H), 1,45 (m, 2H), 1,6-2,0 (massif, 4H), 2,20 (m, 2H), 3,0 (t, 2H).

1.3. 5-butyl-6-[[2-[2-[1(ou 2)-(triphénylméthyl)-1(ou 2)*H*-tétrazol-5-yl]phényl]quinoléin-6-yl]méthyl]-4,6-diazaspiro[2.4] hept-4-èn-7-one

A 0,35 g (1,73 mmoles) du chlorhydrate obtenu à l'étape 1.2, on ajoute 1 g (7,24 mmoles) de carbonate de potassium et 1,42 g (1,87 mmoles) du composé obtenu précédemment dans l'étape 1.1, pur à 80 % et 6 ml de diméthylformamide. On laisse sous agitation, à la température ambiante, pendant une nuit. On reprend le milieu réactionnel par de l'eau et on l'extrait par du dichlorométhane. On récupère la phase organique, on évapore le solvant et on sèche sur du sulfate de magnésium. On purifie le résidu par chromatographie sur colonne de gel de silice en éluant par un mélange toluène/acétate d'éthyle (80/20).

1.4. 5-butyl-6-[[2-[2-(1*H*-tétrazol-5-yl)phényl]quinoléin-6-yl]méthyl]-4,6-diazaspiro[2.4]hept-4-èn-7-one

On chauffe, pendant 5 heures, à la température de reflux, 0,5 g du composé obtenu dans l'étape précédente en solution dans 20 ml d'un mélange acide acétique/méthanol (90/10). On évapore les solvants et on obtient une gomme que l'on purifie par chromatographie sur colonne de gel de silice en éluant par un mélange acétate d'éthyle/méthanol/acide acétique (100/5/0,5). On cristallise le produit dans l'eau.

RMN $^1$H (200 MHz, $CDCl_3$): d 0,77 (t, 3H), 1,17-1,4 (m, 2H), 1,42-1,75 (m, 6H), 2,5 (t, 2H), 4,98 (s, 2H), 7,5-8

(m, 8H), 8,3 (d, 1H), 16,2-16,6 (massif, 1H).

1.5. 5-butyl-6-[[2-[2-(1*H*-tétrazol-5-yl)phényl]quinoléin-6-yl]méthyl)-4,6-diazaspiro[2.4]hept-4-èn-7-one, dichlorhydrate

On dissout le composé obtenu dans l'étape 1.3 dans un volume minimal d'éther, on ajoute 2 équivalents d'acide chlorhydrique 4 N et on agite le mélange pendant une nuit à la température ambiante. Le chlorhydrate cristallise dans le milieu réactionnel. On le recueille par filtration et on le lave à l'éther.
Point de fusion = 128 °C (décomposition)

Exemple 2

2-butyl-3-[[2-[2-(1*H*-tétrazol-5-yl)phényl]quinoléin-6-yl]méthyl]-1,3-diazaspiro[4.4]non-1-èn-4-one

2.1. 2-butyl-3-[[2-[2-[1(ou 2)-(triphénylméthyl)-1(ou 2)*H*-tétrazol-5-yl]phényl]quinoléin-6-yl]méthyl]-1,3-diazaspiro[4.4]non-1-èn-4-one

2.1.1. chlorhydrate de 2-butyl-1,3-diazaspiro[4.4]non-1-èn-4-one
On prépare le chlorhydrate de 2-butyl-1,3-diazaspiro[4.4]non-1-èn-4-one selon la méthode décrite dans l'étape 1.2 à partir du 1-amino-1-cyclopentylcarboxylate d'éthyle.
2.1.2. 2-butyl-3-[[2-[2-[1(ou 2)-(triphénylméthyl)-l(ou 2)*H*-tétrazol-5-yl]phényl]quinoléin-6-yl]méthyl]-1,3-diazaspiro[4.4]non-1-èn-4-one
A 0,35 g (1,52 mmoles) du chlorhydrate obtenu dans l'étape précédente, en solution dans 6 ml de diméthylformamide, on ajoute 1 g (7,24 mmoles) de carbonate de potassium et 1,25 g (1,64 mmoles) du composé obtenu dans l'étape 1.1, pur à 80 %. On laisse sous agitation, à la température ambiante, pendant une nuit. On reprend le milieu réactionnel par de l'eau et on l'extrait par du toluène. On récupère la phase organique, on évapore le solvant et on la sèche sur du sulfate de magnésium. On purifie le résidu par chromatographie sur colonne de gel de silice en éluant par un mélange toluène/acétate d'éthyle (85/15).
On obtient 0,8 g du produit attendu.

2.2. 2-butyl-3-[[2-[2-(1*H*-tétrazol-5-yl)phényl]quinoléin-6-yl]méthyl]-1,3-diazaspiro[4.4]non-1-èn-4-one

On chauffe à la température de reflux, pendant 6 heures, 20 ml d'un mélange méthanol/acide acétique (90/10) contenant 0,8 g du composé obtenu précédemment dans l'étape 2.1. On élimine les solvants sous pression réduite et on cristallise le résidu dans l'éther.
On obtient 0,3 g de produit.
RMN $^1H$ (200 MHz, $CDCl_3$): d 0,8 (t, 3H), 1,17-1,37 (m, 2H), 1,38-1,62 (m, 2H), 1,62-2 (m, 8H), 2,4 (t, 2H), 4,95 (s, 2H), 7,55 (d, 2H), 7,6-8 (m, 6H), 3,5(d, 1H), 16,2-16,6(massif, 1H).

Exemple 3

2-butyl-3-[[2-[2-(1*H*-tétrazol-5-yl)phényl]quinoléin-6-yl]méthyl]-1,3-diazaspiro[4,5]déc-1-èn-4-one

3.1. 2-butyl-3-[[2-[2-[1(ou 2)-(triphénylméthyl)-1(ou 2)*H*-tétrazol-5-yl)phényl]quinoléin-6-yl]méthyl]-1,3-diazaspiro[4,5]déc-1-èn-4-one

3.1.1. 2-butyl-1,3-diazaspiro[4,5]déc-1-èn-4-one
On chauffe à la température de reflux pendant 8 heures, un mélange de 11,3 g (8,8 mmoles) de pentanimidate d'éthyle et 11,56 g (6,7 mmoles) de 1-amino-1-cyclohexane carboxylate d'éthyle, dans 100 ml de xylène contenant 0,6 ml d'acide acétique. On laisse refroidir le mélange, on élimine l'insoluble et on évapore le solvant sous pression réduite. On recristallise le produit dans l'éther.
Point de fusion = 124-125 °C
3.1.2. 2-butyl-3-[[2-[2-[1(ou 2)-(triphénylméthyl)-1(ou 2)*H*-tétrazol-5-yl)phényl]quinoléin-6-yl]méthyl]-1,3-diazaspiro[4,5]déc-1-èn-4-one
A 25 ml de *N,N*-diméthylformamide, on ajoute 1,64 g (7,88 mmoles) du composé obtenu dans l'étape précédente, 3 g (21,7 mmoles) de carbonate de potassium et 5 g (7,47 mmoles) du composé obtenu dans l'exemple 1.1 pur à 90 %. On agite le mélange pendant une nuit à la température ambiante et on le verse dans 100 ml d'eau. On filtre et on purifie le résidu par chromatographie sur colonne de gel de silice en

éluant par un mélange toluène/acétate d'éthyle (80/20).
On obtient 2,3 g de produit sous forme d'une gomme.

3.2. 2-butyl-3-[[2-[2-(1*H*-tétrazol-5-yl)phényl]quinoléin-6-yl]méthyl]-1,3-diazaspiro[4,5]déc-1-èn-4-one

On chauffe à la température de reflux pendant 5 heures, 0,59 g (0,8 mmoles) du composé obtenu dans l'étape précédente dans 20 ml d'un mélange méthanol/acide acétique (90/10). On évapore les solvants sous vide et on cristallise le résidu dans l'éther.
On obtient 0,2 g de produit.
Point de fusion = 122-123 °C
RMN $^1$H (200 MHz, $CDCl_3$): d 0,8(t,3H), 1,17-1,37(m,2H), 1,37-1,88 (massif,12H), 2,4(t,2H), 4,9(s,2H), 7,5(d,2H), 7,6-8(m,6H), 8,35(d,1H)

Le tableau suivant illustre les structures et les propriétés physiques de quelques composés selon l'invention.

Tableau

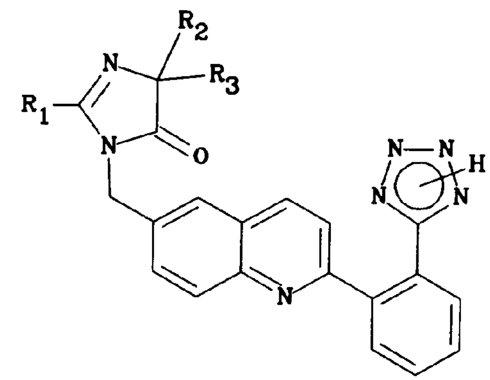

| No | $R_1$ | $R_2$ | $R_3$ | Sel | F (°C) | Données RMN |
|---|---|---|---|---|---|---|
| 1 | $-(CH_2)_3CH_3$ | $-CH_3$ | $-CH_3$ | - | | d 0,77(t,3H), 1,15-1,42(m,8H), 1,5(m,2H), 2,38(m,2H), 4,9(s,2H), 7,6(d,2H), 7,6-8(m,6H), 8,37(d,1H), 16,2-16,6(massif,1H) |
| 2 | $-(CH_2)_3CH_3$ | $-CH_2CH_3$ | $-CH_2CH_3$ | - | | d 0,62(t,6H), 0,85(t,3H), 1,22-1,42(m,2H), 1,45-1,6(m,2H), 1,67(q,4H), 2,48(t,2H), 4,9(s,2H), 7,5-8(m,8H), 8,3(d,1H) |
| 3 | $-(CH_2)_3CH_3$ | $-(CH_2)_2-$ | | - | | d 0,77(t,3H), 1,17-1,4(m,2H), 1,42-1,75(m,6H), 2,5(t,2H), 4,98(s,2H), 7,5-8 (m,8H), 8,3(d,1H), 16,2-16,6(massif,1H) |

| No | $R_1$ | $R_2$ | $R_3$ | Sel | F (°C) | Données RMN |
|---|---|---|---|---|---|---|
| 4 | $-(CH_2)_3CH_3$ | $-(CH_2)_2-$ | | 2 HCl | 128 (déc) | |
| 5 | $-(CH_2)_3CH_3$ | $-(CH_2)_4-$ | | - | | d 0,8(t,3H), 1,17-1,37(m,2H), 1,38-1,62 (m,2H), 1,62-2(m,8H), 2,4(t,2H), 4,95(s,2H), 7,55(d,2H), 7,6-8(m,6H), 3,5(d,1H), 16,2-16,6(massif,1H) |
| 6 | $-(CH_2)_3CH_3$ | $-(CH_2)_5-$ | | - | 122-123 | d 0,8(t,3H), 1,17-1,37(m,2H), 1,37-1,88 (massif,12H), 2,4(t,2H), 4,9(s,2H), 7,5(d,2H), 7,6-8(m,6H), 8,35(d,1H) |

<u>Légende du tableau</u>

dans la colonne "Sel"
2 HCl représente le dichlorhydrate

dans la colonne "F(°C)"
"déc" signifie décomposition

RMN $^1H$ mesurée à 200 MHz dans le diméthylsulfoxyde.

Les composés de l'invention ont fait l'objet d'études pharmacologiques qui ont mis en évidence leurs propriétés antagonistes de l'angiotensine II.

Essai de liaison (binding) de [$^3$H]-angiotensine II sur la corticosurrénale de lapin.

On utilise des lapins mâles Fauves de Bourgogne de 2 à 3 kg. Après sacrifice par dislocation cervicale, on excise les glandes surrénales et on dissèque le cortex sur une boite de culture refroidie par de la glace. On le place dans 10 ml d'une solution tampon glacée à 10 mM de tris(hydroxyméthyl)aminométhane, contenant 0,33 M de saccharose et 1 mM d'acide éthylènediaminetétraacétique et dont le pH a été ajusté à 7,4 avec de l'acide chlorhydrique. On homogénéise le tissu à l'aide d'un appareil Potter électrique par 13 allers et retours de piston à la vitesse de 1200 tours par minute. On ajuste le volume de la préparation à 25 ml avec du tampon Tris-saccharose avant de centrifuger 15 min à 1075 x g. Le surnageant est conservé. Le culot est à nouveau homogénéisé, après remise en suspension dans 10 ml de tampon Tris-saccharose, par passage au Potter électrique puis centrifugé dans les conditions décrites précédemment. Le surnageant obtenu est ajouté au premier, et ils sont centrifugés 30 min à 47 800 x g. On reprend finalement les culots par 150 volumes (soit 100 mg de tissu dans 15 ml de tampon) d'une solution tampon de Tris-HCl à 50 mM, contenant 150 mM de NaCl, 5 mM d'acide éthylènediaminetétraacétique, 1,25 µg/ml de bacitracine, 100 µM de fluorure de phényl-méthylsulfonyle et 0,2 % de sérum albumine bovine (pH = 7,4 à 25°C).
Cette suspension contient les microsomes de corticosurrénales et sera utilisée telle quelle dans les études décrites ci-dessous.
On fait incuber des fractions aliquotes de 100 µl de suspension, en présence de [$^3$H]-angiotensine II (New England Nuclear, d'une activité spécifique de 61 Ci/mmole) dans un volume final de 1 ml de tampon Tris-HCl dont la composition a été précédemment décrite. Après 30 minutes d'incubation à 25°C, on récupère les microsomes par filtration sur filtres de nitrate de cellulose Millipore HAWP™ 0,45 µm préalablement conditionnés par trempage dans une solution à 1 % de sérum albumine bovine. Les filtres sont lavés trois fois avec 5 ml de tampon Tris-HCl glacé. La quantité de radioactivité liée au tissu et retenue sur les filtres est mesurée par spectrométrie de scintillation.

La liaison non spécifique de la [$^3$H]-angiotensine II est mesurée par incubation en présence de 1 µM d'angiotensine II non radioactive. Cette liaison non spécifique représente 5 à 10 % de la quantité totale de radioactivité fixée sur le filtre. La liaison spécifique est la différence entre la radioactivité totale recueillie sur le filtre et la radioactivité non spécifique. On mesure la liaison de la [$^3$H]-angiotensine en présence de différentes concentrations de composés à étudier et on détermine graphiquement la $CI_{50}$, concentration du composé étudié qui inhibe 50 % de la liaison spécifique de la [$^3$H]-angiotensine II.
Les $CI_{50}$ des composés de l'invention sont inférieures à 50 nM.

Inhibition de la réponse à l'angiotensine II sur la pression artérielle de rat.

On utilise des rats mâles (Sprague-Dawley, Charles River France) pesant 250 à 280 g, que l'on anesthésie au pentobarbital sodique (55 mg/kg i.p.) et que l'on maintient sous respiration artificielle (Respirateur Harvard™; fréquence de respiration de 70 ml par minute, volume d'air 1 ml par 100 g de poids corporel). On "spinalise" les animaux à l'aide d'une tige métallique, introduite par l'orbite de l'oeil droit et descendue le long de la colonne vertébrale. On sectionne les nerfs vagues droit et gauche (bivagotomie); on ligature l'artère carotide droite, l'artère carotide gauche étant cathétérisée afin de mesurer la pression artérielle à l'aide d'une cellule de pression (type Statham™ P23Db). On cathétérise une veine fémorale en vue de l'administration de divers composés.
On mesure les variations de pression artérielle moyenne induites par l'angiotensine administrée par voie intraveineuse à la dose de 0,5 µg/kg avant l'administration des composés de l'invention et celles induites par l'angiotensine administrée dans les mêmes conditions 5 minutes après l'administration par voie intraveineuse des composés de l'invention ou 30 minutes après leur administration par voie orale. Les composés de l'invention sont administrés à des doses allant de 0,01 à 100 mg/kg.
Le pourcentage d'inhibition de la réponse contrôle à l'angiotensine II est utilisé pour apprécier le potentiel antagoniste à l'angiotensine II des composés de l'invention.

Les composés de l'invention ou leurs sels appropriés peuvent être utilisés pour le traitement de diverses formes de pathologies hypertensives et d'insuffisances cardiaque, rénale ou pulmonaire ainsi que pour le traitement du glaucome.

Les composés de l'invention ou leurs sels appropriés peuvent également être utilisés en association avec d'autres substances à activité cardiovasculaire, telles que les diurétiques, les α-bloquants, les β-bloquants, les antagonistes calciques ou les inhibiteurs de l'enzyme de conversion de l'angiotensine I.

Les composés de l'invention ou leurs sels appropriés peuvent être présentés sous toutes formes pharmaceutiques appropriées au traitement pour l'administration orale, parentérale, intramusculaire ou rectale : comprimés, capsules, gélules, solutions ou suspensions stériles, suppositoires etc...

Pour le traitement du glaucome, les composés de l'invention peuvent être présentés sous la forme de comprimés, gélules, solutions injectables ou formulations oculaires topiques.

Les composés de l'invention peuvent être administrés aux patients en une quantité pouvant aller de 1 à 1000 mg par jour et par patient, en une ou plusieurs doses.

## Revendications

1. Dérivés de 3-(quinoléin-6-yl-méthyl)-4*H*-imidazol-4-one répondant à la formule (I)

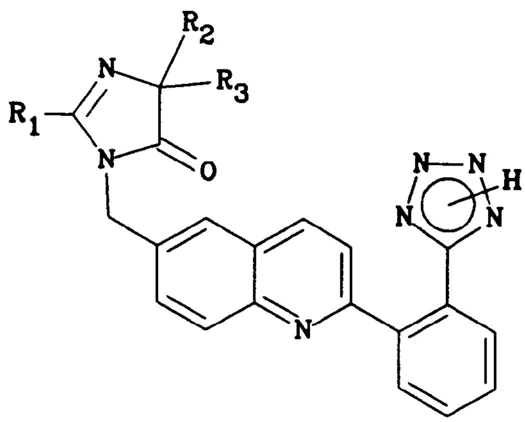

(I)

dans laquelle
$R_1$ représente un groupe ($C_2$-$C_5$)alkyle droit ou ramifié,
$R_2$ et $R_3$ représentent, chacun indépendamment l'un de l'autre, soit un atome d'hydrogène, soit un groupe ($C_1$-$C_5$)alkyle droit ou ramifié, soit un groupe $(CH_2)_n$-aryle où n = 0 à 3,
ou $R_2$ et $R_3$ peuvent former avec le cycle imidazole un groupe spirocyclo($C_3$-$C_8$)alkyle,
ainsi que leurs sels d'addition aux acides et aux bases pharmaceutiquement acceptables.

2. Dérivés selon la revendication 1 caractérisés en ce que
$R_1$ représente un groupe ($C_2$-$C_5$)alkyle droit ou ramifié,
$R_2$ et $R_3$ représentent, chacun indépendamment l'un de l'autre, un groupe ($C_1$-$C_5$)alkyle droit ou ramifié,
ou $R_2$ et $R_3$ peuvent former avec le cycle imidazole un groupe spirocyclo($C_3$-$C_8$)alkyle.

3. Dérivés selon la revendication 2 caractérisés en ce que
$R_1$ représente un groupe butyle et
$R_2$ et $R_3$ représentent, chacun indépendamment l'un de l'autre, un groupe ($C_1$-$C_5$)alkyle droit ou ramifié,
ou $R_2$ et $R_3$ peuvent former avec le cycle imidazole un groupe spirocyclo($C_3$-$C_8$)alkyle.

4. La 5-butyl-6-[[2-[2-(1*H*-tétrazol-5-yl]phényl)quinoléin-6-yl]méthyl]-4,6-diazaspiro[2.4]hept-4-èn-7-one.

5. La 2-butyl-3-[[2-[2-(1*H*-tétrazol-5-yl)phényl]quinoléin-6- yl]méthyl]-1,3-diazaspiro[4.4]non-1-èn-4-one.

6. La 2-butyl-3-[[2-[2-(1*H*-tétrazol-5-yl)phényl]quinoléin-6-yl]méthyl]-1,3-diazaspiro[4,5]déc-1-èn-4-one

7. Procédé de préparation des dérivés selon la revendication 1, procédé caractérisé en ce que l'on fait réagir un composé de formule (VII)

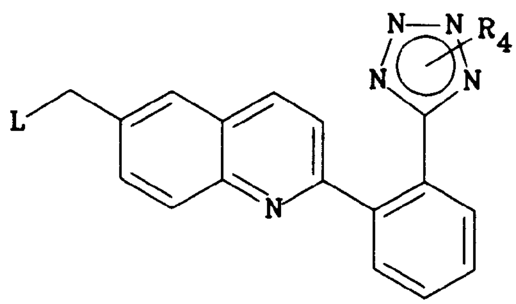

(VII)

dans laquelle L représente un groupe partant et $R_4$ représente un groupe de formule $CR_5R_5R_7$ où $R_5$, $R_5$ et $R_7$ sont chacun indépendamment l'un de l'autre un groupe $(C_1\text{-}C_2)$alkyle ou un groupe aryle, avec une imidazolone de formule (VIII)

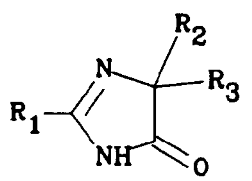

(VIII)

dans laquelle $R_1$ représente un groupe $(C_2\text{-}C_5)$alkyle droit ou ramifié, $R_2$ et $R_3$ représentent, chacun indépendamment l'un de l'autre, soit un atome d'hydrogène, soit un groupe $(C_1\text{-}C_5)$alkyle droit ou ramifié, soit un groupe $(CH_2)_n$-aryle où n = 0 à 3 ou $R_2$ et $R_3$ peuvent former avec le cycle imidazole un groupe spirocyclo$(C_3\text{-}C_8)$alkyle,
pour obtenir un composé de formule (IX)

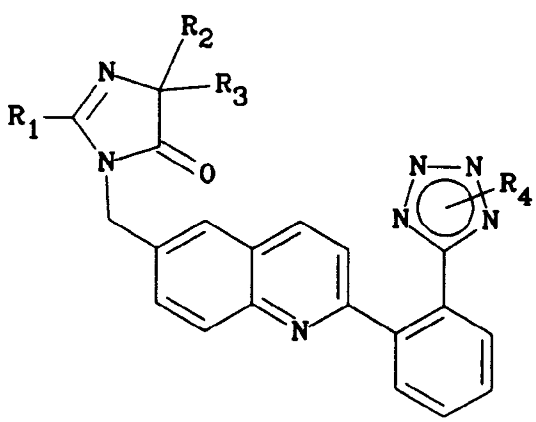

(IX)

que l'on déprotège.

**8.** Médicament caractérisé en ce qu'il contient un composé selon l'une quelconque des revendications 1 à 6.

**9.** Composition pharmaceutique caractérisée en ce qu'elle contient un composé selon l'une quelconque des revendications 1 à 6, en association avec tout excipient approprié .

**10.** Dérivés répondant à la formule (IX)

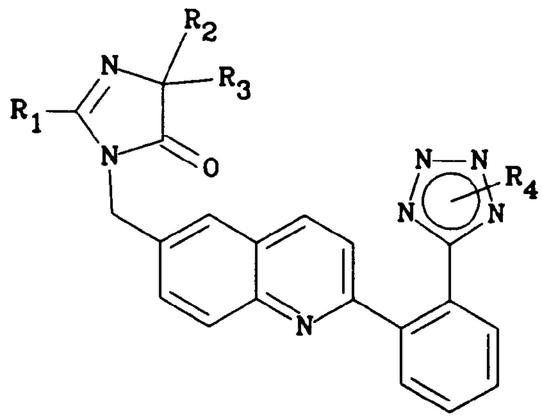

(IX)

dans laquelle

$R_1$ représente un groupe $(C_2-C_5)$alkyle droit ou ramifié,

$R_2$ et $R_3$ représentent, chacun indépendamment l'un de l'autre, soit un atome d'hydrogène, soit un groupe $(C_1-C_5)$alkyle droit ou ramifié, soit un groupe $(CH_2)_n$-aryle où n = 0 à 3

ou $R_2$ et $R_3$ peuvent former avec le cycle imidazole un groupe spirocyclo$(C_3-C_8)$alkyle et

$R_4$ représente un groupe de formule $CR_5R_6R_7$ où $R_5$, $R_5$ et $R_7$ sont chacun indépendamment l'un de l'autre un groupe $(C_1-C_2)$alkyle ou un groupe aryle,

dérivés utiles comme intermédiaires pour la synthèse des dérivés selon la revendication 1.

Office européen des brevets

# RAPPORT DE RECHERCHE EUROPEENNE

Numero de la demande
EP 93 40 2949

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| A | JOURNAL OF MEDICINAL CHEMISTRY.<br>vol. 33, no. 5 , 1990 , WASHINGTON US<br>pages 1330 - 1336<br>D.J. CARINI ET AL. 'Nonpeptide angiotensin II antagonist'<br>* page 1330, formule 1 *<br>--- | 1,8 | C07D401/14<br>A61K31/47 |
| D,P,A | EP-A-0 540 400 (SYNTHELABO)<br>* revendications *<br>--- | 1,8 | |
| P,A | EP-A-0 569 013 (THE GREEN CROSS CORPORATION)<br>* revendications *<br>--- | 1,8 | |
| P,A | JOURNAL OF MEDICINAL CHEMISTRY<br>vol. 36, no. 22 , 29 Octobre 1993 , WASHINGTON US<br>pages 3371 - 3380<br>CL. A. BERNART ET AL. 'A new series of imidazolones: highly specific and potent nonpeptide AT1 angiotensin II receptor antagonists'<br>* document complet *<br>----- | 1,8 | DOMAINES TECHNIQUES RECHERCHES (Int.Cl.5)<br>C07D |

Le présent rapport a été établi pour toutes les revendications

| Lieu de la recherche | Date d'achèvement de la recherche | Examinateur |
|---|---|---|
| LA HAYE | 22 Mars 1994 | Van Bijlen, H |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)